# EUROPÄISCHE PATENTSCHRIFT

(11) **EP 0 682 943 B1**
(45) Veröffentlichungstag und Bekanntmachung des Hinweises auf die Patenterteilung: **20.08.2003**
(21) Anmeldenummer: 95107739.5
(22) Anmeldetag: 19.05.1995
(51) Int. Cl.: A61K 9/107, A61K 31/55

(54) **Lormetazepamhaltige Öl-in-Wasser-Emulsion**
Oil-in-water emulsion containing lormetazepam
Emulsion d'huile dans l'eau contenant du lormetazepam

(30) Priorität: 20.05.1994 DE 4417847; 12.09.1994 DE 4432440
(43) Veröffentlichungstag der Anmeldung: 22.11.1995
(73) Patentinhaber: GESELLSCHAFT FÜR MEDIZINISCHE FORSCHUNG UND WEITERBILDUNG e.V. GMF, D-80707 München (DE)
(72) Erfinder:
(74) Vertreter: Laufhütte, Dieter, Dr.-Ing.

(56) Entgegenhaltungen:
- EP-A- 0 011 745
- EP-A- 0 143 305
- EP-A- 0 276 735
- EP-A- 0 391 369
- DE-A- 2 730 570
- BR. J. ANAESTH. (ENGLAND), Bd. 53, Nr. 12, 1981 Seiten 1265-1268, MATTILA M.A.K. ET AL 'Reduction of venous sequelae of i.v. diazepam with a fat emulsion as solvent'
- DEUTSCHE APOTHEKER ZEITUNG, Bd. 135, Nr. 28, 13.Juli 1995 Seiten 2597-2601, MÜLLER R.H. ET AL 'Neue Arzneimittel mit Nanopartikeln'

## Beschreibung

Die Erfindung betrifft eine lormetazepamhaltige therapeutische Zusammensetzung und ein Verfahren zu ihrer Herstellung.

Lorazepam (INN) ist ein 7-chloro-5-(o-chlorophenyl)-1,3-dihydro-3-hydroxy-2H-1,4-benzodiazepin-2-one (WHO) mit der Summenformel (C₁₅H₁₀Cl₂N₂O₂).

Lormetazepam (INN) ist ein 7-chloro-5-(o-chlorophenyl)-1,3-dihydroxy-3-hydro-1-methyl-2H-1,4-benzodiazepin-2-one mit der Summenformel (C₁₆H₁₂Cl₂N₂O₂).

Intravenös und intramuskulär verabreichte lorazepamhaltige therapeutische Zusammensetzungen sind seit 1988 in Deutschland zur Behandlung zugelassen zur Basissedierung vor und während diagnostischer und operativer Eingriffe, einschließlich der Erzeugung amnestischer Effekte, zur Behandlungseinleitung schwerer neurotischer Angstsymptomatik und ausgeprägter Phobien, zur adjuvanten Behandlung schwerer Angst- und Erregungszustände bei Psychosen und Depressionen (bei unzureichender Wirksamkeit der neuroleptischen bzw. antidepressiven Basistherapie) und zur Behandlung des Status epilepticus.

Intravenös verabreichte lormetazepamhaltige therapeutische Zusammensetzungen sind seit 1980 zur Behandlung von akuten Angstzustanden, zur präoperativen Anxiolyse, zur Prämedikation von Narkosen bei diagnostischen und chirurgischen Eingriffen und zur Sedierung in der Intensivmedizin zugelassen.

Einer breiten Anwendung im operativen und intensivmedizinischen Bereich steht jedoch die schlechte Venenverträglichkeit und die Hämo-Inkompatibilität (Unverträglichkeit für die roten Blutkörperchen) im Wege. Zur Langzeitsedierung von Intensivpatienten wird das Präparat trotz grundsätzlicher Vorteile des Wirkstoffs (starke Anxiolyse, retrograde Amnesie, keine Beeinträchtigung des Schlafmusters) praktisch nicht verwendet. Die Ursache der schlechten Venenverträglichkeit der parenteralen Lösung liegt in dem zur Verbesserung der Löslichkeit von Lorazepam bzw. Lormetazepam benutzten Solvent Propylenglykol (PG). Dieses führt in der verwendeten Konzentration von 83% (Lorazepam) bzw.50% (Lormetazepam) zu einer unphysiologisch hohen Osmolalität der Injektionslösung. Durch die hohe Osmolalität kommt es bei der Injektion der lorazepam- bzw. lormetazepamhaltigen therapeutischen Zusammensetzung regelmäßig zur Auflösung von roten Blutkörperchen (Hämolyse).

Bei mehrfacher Anwendung oder Applikation größerer Mengen Propylenglycol kommt es einmal zum Anstieg der Blut-Osmolalität in den unphysiologischen Bereich, zum anderen durch die starke Hämolyse zum Verbrauch der Bindungskapazität für das aus den roten Blutkörperchen freigesetzte Hämoglobin. Dieses wird primär an Haptogolobin gebunden, der Hämoglobin-Haptoglobin Komplex wird im retikuloendothelialen System abgebaut und es resultiert schließlich Bilirubin aus dem Abbau des Hämoglobins. Ist die Haptoglobinbindungskapazität erschöpft, zirkuliert freies Hämoglobin im Plasma. Bei anhaltendem Prozess wird freies Plasma-Hämoglobin in der Niere glomerulär filtriert. Durch Reabsorption des Hämoglobins im Nieren-Tubulus kommt es hier zur Einlagerung von Ferritin und Hämosiderin, wodurch bei Überladung mit diesem aus dem Häm stammenden Eisen schlußendlich die Tubulusepithelien degenerieren und abschilfern. Hierdurch wird die Niere geschädigt.

Aus der EP-A-0 391 369 ist bereits eine lorazepamhaltige O/W-Emulsion zur intravenösen Verabreichung bekannt, bei der der pH-Wert bevorzugt bei 8 liegt.

Aufgabe der Erfindung ist es, eine lormetazepamhaltige therapeutische Zusammensetzung an die Hand zu geben, die bei intravenöser Verabreichung venenverträglich und hämokompatibel ist sowie eine physiologische Osmollalität hat und somit für eine Mehrfach- bzw. Langzeitanwendung (z. B. in der Intensivmedizin) geeignet ist.

Diese Aufgabe wird erfindungsgemäß durch den kennzeichnenden Teil des Hauptanspruchs gelöst. Demnach wird das Lormetazepam in einer Öl-in-Wasser-Emulsion eingesetzt.

Die Öl-in-Wasser-Emulsion besteht aus einer Mischung von Sojabohnenöl, Glycerol und Eigelb-Phospholipiden mit einer für Injektionszwecke ausreichenden Wassermenge.

Die mittlere Tröpfchengröße der Emulsion beträgt ca. 0,5 µm. Der pH-Wert der Lösung kann vorteilhaft au ca. 7,0 bis ca. 8,0 eingestellt sein. Gemäß eines bevorzugten Herstellungsverfahrens lässt sich die erfindungsgemäße lormetazepamhaltige therapeutische Zusammensetzung dadurch herstellen, dass eine Emulsion aus Sojabohnenöl, Glycerol und Eigelb-Phospholipid mit einer ausreichenden Menge Wasser für Injektionszwecke derart gebildet wird, daß sich eine mittlere Tropfengröße der emulgierten Phase von 0,5 µm ergibt. Der pH-Wert dieser Emulsion wird mittels einer Natriumhydroxid-Lösung auf 7,0 bis 8,0 eingestellt. In einem getrennten Ansatz wird Lorazepam bzw. Lormetazepam als mikronisiertes Pulver in Ethanol oder einem anderen Alkohol, wie z.B. Polyethylenglykol, suspendiert. Diese Lormetazepam-Ethanol-Suspension wird mit der öl-in-Wasser-Emulsion gemischt und es wird anschließend so viel Wasser hinzugegeben, daß eine injizierbare Lösung erhalten wird.

Die Öl-in-Wasser-Emulsion läßt sich vorteilhaft in einem Hochdruck-Prozeßbehälter unter Stickstoffatmosphäre bei ca. 20-30° C sehr einfach durch mechanischen Leistungseintrag, beispielsweise Rühren, herstellen.

Die Öl-in-Wasser-Emulsion kann nach dem letzten Mischschritt gefiltert werden, um Partikel von über 1,0 µm Größe abzutrennen.

Ein alternatives Herstellungsverfahren kann darin bestehen, daß Lormetazepam als mikronisiertes Pulver in Ethanol suspendiert wird und daß diese Lormetazepam-Ethanol-Suspension mit einer bereits als Handelsware vorliegenden Öl-in-Wasser-Emulsion vermischt wird. Derartige handelsüblichen Öl-in-Wasser-Emulsionen werden beispielsweise von der Firma Abbott unter der Bezeichnung "ABBOLIPID", von der Firma Pfrimmer Kabi unter der Bezeichnung "INTRALIPID"/"INTRA-LIPID Novum", von der Firma Braun Melsungen unter der Bezeichnung "LIPOFUNDIN"/"LIPOFUNDIN MCT", von der Firma Fresenius unter der Bezeichnung "LIPOVENÖS"/LIPOVENÖS PLR", von der Clintec Salvia unter der Bezeichnung "SALVILIPID", von der Firma Schiwa unter der Bezeichnung "SCHIWALIPID" in den Handel gebracht. Diese handelsüblichen Öl-in-Wasser-Emulsionen werden in umfangreichem Maße zur parenteralen Ernährung von Patienten eingesetzt.

Für die Einschleusung des Lormetazepams in die Fetttröpfchen der Öl-in-Wasser-Emulsion scheint es von großer Bedeutung zu sein, daß sich das Lormetazepam der hergestellten Lormetazepam-Ethanol-Suspension noch nicht vollständig im Ethanol gelöst hat. Vergleichsversuche haben ergeben, daß es wesentlich kritischer ist, vollständig in Ethanol oder in einem anderen geeigneten Lösungsmittel, wie z.B. Polyethylenglykol, gelöstes Lormetazepam mit der Öl-in-Wasser-Emulsion soweit zu mischen, daß eine injizierbare Lösung erhalten wird.

Die Erfindung wird nachfolgend anhand von Beispielen näher erläutert.

### Beispiel 1:

Um eine Lormetazepam-Injektion mit 2 mg zu erhalten, werden Ampullen mit 10 ml der lormetazepamhaltigen therapeutischen Zusammensetzung mit einem Wirkstoffanteil von 0,2 mg/ml Lormetazepam gefüllt.

Ein chargenweiser Herstellungsprozeß zur Herstellung von 10.000 Ampullen mit einer Charge bedingt demgemäß eine Chargengröße von 100 l.

In einem entsprechend großen sterilisierten Hochdruck-Prozeßbehälter mit Rührvorrichtung wird unter Stickstoffatmosphäre eine Emulsion von sterilisiertem
- Sojabohnenöl (z.B. USP, BP, DAB) 20,000 kg
- Glycerol (z.B. USP, BP, DAB) 2,250 kg
- Eigelb Phospolipide 1,200 kg
mit einer ausreichenden Menge Wasser für Injektionszwecke so lange gerührt, bis eine Partikelgröße von 0,5 µm erzielt wird. Der Druck im Hochdruck-Prozeßbehälter wird dabei maximal auf 10 bar eingestellt und die Temperatur kann auf ca. 20 bis ca. 30° C eingestellt werden.

Der pH-Wert wird durch eine sterile Natriumhydroxid(z.B. USP, BP, DAB)-Lösung auf 7,0 bis 8,0 eingestellt.

In einem getrennten hitzesterilisierten Rührkessel wird eine Suspension aus sterilisiertem

| | |
|---|---|
| Lormetazepam (als mikronisiertes Pulver) | 0,020 kg |
| in Ethanol (z.B. USP, BP, DAB) | 1,000 kg |

hergestellt.

Beide Ansätze werden miteinander vermischt und zur Homogenisierung für eine ausreichende Zeit gerührt.

Anschließend wird unter Rühren mit Wasser auf die Chargengröße von 100 l aufgefüllt.

Danach wird die Emulsion gefiltert, um Partikel von über 1,0 µm Größe abzutrennen.

Schließlich wird die Emulsion unter Stickstoff-Atmosphäre und aseptischen Bedingungen in sterile braune Glasampullen abgefüllt, wobei das Abfüllvolumen der Ampullen jeweils 10,0 bis 10,2 ml Emulsion beträgt.

### Beispiel 2:

In einem hitzesterilisierten Rührkessel wird eine Suspension aus sterilisiertem

| | |
|---|---|
| Lormetazepam (als mikronisiertes Pulver) | 0,020 kg |
| in Ethanol (z.B. USP, BP, DAB) | 1,000 kg |

hergestellt.

In einem sterilisierten Prozeßbehälter wird eine Menge ABBO-LIPID-Emulsion (10%ig bzw. 20%ig) der Firma Abbott unter sterilen Bedingungen eingefüllt, so daß eine Chargengröße von 100 l erreicht wird.

Die vorbereitete Suspension wird in den sterilisierten Prozeßbehälter hinzugegeben und so lange gerührt, bis sich eine weitgehend homogene therapeutische Zusammensetzung ergibt.

### Beispiel 3:

In einem hitzesterilisierten Rührkessel wird eine Suspension aus sterilisiertem

| | |
|---|---|
| Lormetazepam (als mikronisiertes Pulver) | 0,020 kg |
| in Ethanol (z.B. USP, BP, DAB) | 1,000 kg |

hergestellt.

In einem sterilisierten Prozeßbehälter wird eine Menge LIPO-FUNDIN-Emulsion (10%ig/20%ig) der Firma Braun Melsungen unter sterilen Bedingungen eingefüllt, so daß eine Chargengröße von 100 l erreicht wird.

Die vorbereitete Suspension wird in dem sterilisierten Prozeßbehälter hinzugegeben und so lange gerührt, bis sich eine weitgehend homogene therapeutische Zusammensetzung ergibt.

Anschließend wird das Volumen im Prozeßbehälter mit Wasser auf 100 l aufgefüllt, um eine injizierbare therapeutische Zusammensetzung zu schaffen.

### Beispiel 4:

In einem hitzesterilisierten Rührkessel wird eine Suspension aus sterilisiertem

| | |
|---|---|
| Lormetazepam (als mikronisiertes Pulver) | 0,020 kg |
| in Ethanol (z.B. USP, BP, DAB) | 1,000 kg |

hergestellt.

In einem sterilisierten Prozeßbehälter wird eine Menge LIPOFUNDIN MCT-Emulsion (10%ig/20%ig) der Firma Braun Melsungen unter sterilen Bedingungen eingefüllt, so daß eine Chargengröße von 100 l erreicht wird.

Die vorbereitete Suspension wird in den sterilisierten Prozeßbehälter hinzugegeben und so lange gerührt, bis sich eine weitgehend homogene therapeutische Zusammensetzung ergibt.

### Beispiel 5:

In einem hitzesterilisierten Rührkessel wird eine Suspension aus sterilisiertem

| | |
|---|---|
| Lormetazepam (als mikronisiertes Pulver) | 0,020 kg |
| in Ethanol (z.B. USP, BP, DAB) | 1,000 kg |

hergestellt.

In einem sterilisierten Prozeßbehälter wird eine Menge LIPO-VENÖS-Emulsion (10%ig/20%ig) bzw. eine LIPOVENÖS PLR-Emulsion der Firma Fresenius unter sterilen Bedingungen eingefüllt, so daß eine Chargengröße von 100 l erreicht wird.

Die vorbereitete Suspension wird in den sterilisierten Prozeßbehälter hinzugegeben und so lange gerührt, bis sich eine weitgehend homogene therapeutische Zusammensetzung ergibt.

### Beispiel 6:

In einem hitzesterilisierten Rührkessel wird eine Suspension aus sterilisiertem

| | |
|---|---|
| Lormetazepam (als mikronisiertes Pulver) | 0,020 kg |
| in Ethanol (z.B. USP, BP, DAB) | 1,000 kg |

hergestellt.

In einem sterilisierten Prozeßbehälter wird eine Menge INTRA-LIPID-Emulsion (10%ig bzw. 20%ig) der Firma Pfrimmer Kabi unter sterilen Bedingungen eingefüllt, so daß eine Chargengröße von 100 l erreicht wird.

Die vorbereitete Suspension wird in den sterilisierten Prozeßbehälter hinzugegeben und so lange gerührt, bis sich eine weitgehend homogene therapeutische Zusammensetzung ergibt.

### Beispiel 7:

In einem hitzesterilisierten Rührkessel wird eine Suspension aus sterilisiertem

| | |
|---|---|
| Lormetazepam (als mikronisiertes Pulver) | 0,020 kg |
| in Ethanol (z.B. USP, BP, DAB) | 1,000 kg |

hergestellt.

In einem sterilisierten Prozeßbehälter wird eine Menge SALVI-LIPID-Emulsion (10% bzw. 20%) der Firma Clintec Salvia unter sterilen Bedingungen eingefüllt, so daß eine Chargengröße von 100 l erreicht wird.

Die vorbereitete Suspension wird in den sterilisierten Prozeßbehälter hinzugegeben und so lange gerührt, bis sich eine homogene therapeutische Zusammensetzung ergibt.

### Beispiel 8 (Vergleichsbeispiel):

Um eine Lorazepam-Injektion mit 2 mg zu erhalten, werden Ampullen mit 5 ml der lorazepamhaltigen therapeutischen Zusammensetzung mit einem Wirkstoffanteil von 0,4 mg/ml Lorazepam gefüllt.

Ein chargenweiser Herstellungsprozeß zur Herstellung von 10.000 Ampullen Lorazepam in einer Charge bedingt demgemäß ein Volumen von 50 l.

In einem entsprechend großen sterilisierten Hochdruck-Prozeßbehälter mit Rührvorrichtung wird unter Stickstoffatmosphäre eine Emulsion von sterilisiertem

| | |
|---|---|
| Sojabohnenöl (z.B. USP, BP, DAB) | 20,000 kg |
| Glycerol (z.B. USP, BP, DAB) | 2,250 kg |
| Eigelb Phospholipide | 1,200 kg |

mit einer ausreichenden Menge Wasser für Injektionszwecke so lange gerührt, bis eine Partikelgröße von 0,5 µm erzielt wird.

Der Druck im Hochdruck-Prozeßbehälter wird dabei maximal auf 10 bar eingestellt und die Temperatur kann auf ca. 20 bis ca. 30°C eingestellt werden.

Der pH-Wert wird durch eine sterile Natriumhydrochlorid-Lösung (z.B. USP, BP, DAB) auf 7,0 bis 8,0 eingestellt.

In einem getrennten hitzesterilisierten Rührkessel wird eine Suspension aus sterilisiertem

| | |
|---|---|
| Lorazepam (als mikronisiertes Pulver) | 0,020 kg |
| in Ethanol (z.B. USP, BP, DAB) | 1,000 kg |

hergestellt.

Beide Ansätze werden miteinander vermischt und zur Homogenisierung für eine ausreichende Zeit gerührt.

Anschließend wird unter Rühren Wasser auf die Chargengröße von 50 l aufgefüllt.

Danach wird die Emulsion gefiltert, um Partikel von über 1,0 µm Größe abzutrennen.

Schließlich wird die Emulsion unter Stickstoff-Atmosphäre und aseptischen Bedingungen in sterile braune Glasampullen abgefüllt, wobei das Abfüllvolumen der Ampullen jeweils 5,0 bis 5,1 ml Emulsion beträgt.

### Beispiel 9 (Vergleichsbeispiel):

In einem hitzesterilisierten Rührkessel wird eine Suspension aus sterilisiertem

| | |
|---|---|
| Lorazepam (als mikronisiertes Pulver) | 0,020 kg |
| in Ethanol (z.B. USP, BP, DAB) | 1,000 kg |

hergestellt.

In einem sterilisierten Prozeßbehälter wird eine ABBOLIPID-Emulsion (10% bzw. 20%) der Firma Abbot unter sterilen Bedingungen eingefüllt, so daß eine Chargengröße von 50 l erreicht wird.

Die vorbereitete Suspension wird in den sterilisierten Prozeßbehälter hinzugegeben und so lange gerührt, bis sich eine homogene therapeutische Zusammensetzung ergibt.

### Beispiel 10 (Vergleichsbeispiel):

In einem hitzesterilisierten Rührkessel wird eine Suspension aus sterilisiertem

| | |
|---|---|
| Lorazepam (als mikronisiertes Pulver) | 0,020 kg |
| in Ethanol (z.B. USP, BP, DAB) | 1,000 kg |

hergestellt.

In einem sterilisierten Prozeßbehälter wird eine LIPOFUNDIN-Emulsion (10% bzw. 20%) bzw. LIPOFUNDIN MCT-Emulsion (10% bzw. 20%) der Firma Braun Melsungen unter sterilen Bedingungen eingefüllt, so daß eine Chargengröße von 50 l erreicht wird.

Die vorbereitete Suspension wird in den sterilisierten Prozeßbehälter hinzugegeben und so lange gerührt, bis sich eine homogene therapeutische Zusammensetzung ergibt.

### Beispiel 11 (Vergleichsbeispiel):

In einem hitzesterilisierten Rührkessel wird eine Suspension aus sterilisiertem

| | |
|---|---|
| Lorazepam (als mikronisiertes Pulver) | 0,020 kg |
| in Ethanol (z.B. USP, BP, DAB) | 1,000 kg |

hergestellt.

In einem sterilisierten Prozeßbehälter wird eine LIPOVENÖS-Emulsion (10% bzw. 20%) bzw. eine LIPOVENÖS PLR-Emulsion (10%) der Firma Fresenius unter sterilen Bedingungen eingefüllt, so daß eine Chargengröße von 50 l erreicht wird.

Die vorbereitete Suspension wird in den sterilisierten Prozeßbehälter hinzugegeben und so lange gerührt, bis sich eine homogene therapeutische Zusammensetzung ergibt.

### Beispiel 12 (Vergleichsbeispiel):

In einem hitzesterilisierten Rührkessel wird eine Suspension aus sterilisiertem

| | |
|---|---|
| Lorazepam (als mikronisiertes Pulver) | 0,020 kg |
| in Ethanol (z.B. USP, BP, DAB) | 1,000 kg |

hergestellt.

In einem sterilisierten Prozeßbehälter wird eine INTRALIPID 10 Novum-Emulsion bzw. eine INTRALIPID 20-Emulsion der Firma Pfrimmer Kabi unter sterilen Bedingungen eingefüllt, so daß eine Chargengröße von 50 l erreicht wird.

Die vorbereitete Suspension wird in den sterilisierten Prozeßbehälter hinzugegeben und so lange gerührt, bis sich eine homogene therapeutische Zusammensetzung ergibt.

### Beispiel 13 (Vergleichsbeispiel):

In einem hitzesterilisierten Rührkessel wird eine Suspension aus sterilisiertem

| | |
|---|---|
| Lorazepam (als mikronisiertes Pulver) | 0,020 kg |
| in Ethanol (z.B. USP, BP, DAB) | 1,000 kg |

hergestellt.

In einem sterilisierten Prozeßbehälter wird eine SCHIWALIPID-Emulsion (10% bzw. 20%) der Firma Schiwa unter sterilen Bedingungen eingefüllt, so daß eine Chargengröße von 50 l erreicht wird.

Die vorbereitete Suspension wird in den sterilisierten Prozeßbehälter hinzugegeben und so lange gerührt, bis sich eine homogene therapeutische Zusammensetzung ergibt.

### Beispiel 14 (Vergleichsbeispiel):

In einem hitzesterilisierten Rührkessel wird eine Suspension aus sterilisiertem

| | |
|---|---|
| Lorazepam (als mikronisiertes Pulver) | 0,020 kg |
| in Ethanol (z.B. USP, BP, DAB) | 1,000 kg |

hergestellt.

In einem sterilisierten Prozeßbehälter wird eine SALVILIPID-Emulsion (10% bzw. 20%) der Firma Clintec Salvia unter sterilen Bedingungen eingefüllt, so daß eine Chargengröße von 50 l erreicht wird.

Die vorbereitete Suspension wird in den sterilisierten Prozeßbehälter hinzugegeben und so lange gerührt, bis sich eine homogene therapeutische Zusammensetzung ergibt.

Die in den Beispielen angegebenen Konzentrationen und Ampullenvolumina können im Rahmen der Erfindung variieren. So kann eine wirksame Konzentration des Lormetazepams im Bereich von 0,2-0,5 mg/ml liegen. Das Ampullenvolumen kann vorteilhaft zwischen 4 und 10 ml variieren.

## Patentansprüche

1. Lormetazepamhaltige therapeutische Zusammensetzung, insbesondere Sedativum, zur intravenösen Verabreichung,
**dadurch gekennzeichnet,**
**daß** Lormetazepam in einer Öl-in-Wasser-Emulsion eingesetzt wird, wobei die Öl-in-Wasser-Emulsion aus einer Mischung von Sojabohnenöl, Glycerol und Eigelb Phospholipiden mit einer ausreichenden Menge an Wasser für Injektionszwecke besteht.

2. Lormetazepamhaltige therapeutische Zusammensetzung, insbesondere Sedativum, nach Anspruch 1, **dadurch gekennzeichnet, dass** die mittlere Tropfengröße der Emulsion ca. 0,5 µm ist.

3. Lormetazepamhaltige therapeutische Zusammensetzung, insbesondere Sedativum, nach Anspruch 1 oder 2, **dadurch gekennzeichnet, dass** der pH-Wert der Lösung auf ca. 7,0 bis ca. 8,0 eingestellt ist.

4. Verfahren zur Herstellung einer lormetazepamhaltigen therapeutischen Zusammensetzung, insbesondere Sedativum, nach einem der Anspruch 1 bis 3, **dadurch gekennzeichnet, dass** eine Emulsion aus Sojabohnen Öl, Glycerol und Eigelb Phospholipid mit einer ausreichenden Menge Wasser für Injektionszwecke derart gebildet wird, dass sich eine mittlere Tropfengröße der emulgierten Phase von 0,5 µm ergibt,
dass der pH-Wert der Emulsion mittels einer Natriumhydroxid-Lösung auf 7,0 bis 8,0 eingestellt wird,
dass in einem getrennten Ansatz Lormetazepam als micronisiertes Pulver in Ethanol oder einen anderen geeigneten Alkohol suspendiert wird,
dass die Lormetazepam-Ethanol-Suspension mit der Öl-in-Wasser Emulsion gemischt wird und dass Wasser hinzugegeben wird, um eine injizierbare Lösung zu erhalten.

5. Verfahren nach Anspruch 4, **dadurch gekennzeichnet, dass** die Öl-in-Wasser-Emulsion in einem Hochdruck-Prozessbehälter unter Stickstoff-Atmosphäre und bei Temperaturen von ca. 20° C bis ca. 30° C hergestellt wird.

6. Verfahren nach Anspruch 4 oder 5, **dadurch gekennzeichnet, dass** die Öl-in-Wasser-Emulsion nach dem letzten Mischschritt gefiltert wird, um Partikel von über 1,0 µm Größe abzutrennen.

## Claims

1. A lormetazepam-containing therapeutic composition, in particular a sedative, for intravenous administration,
**characterised in that**
lormetazepam is used in an oil-in-water emulsion, the oil-in-water emulsion consisting of a mixture of soybean oil, glycerol and egg yolk phospholipids with a quantity of water sufficient for injection purposes.

2. The lormetazepam-containing therapeutic composition, in particular a sedative according to claim 1, **characterised in that** the average droplet size of the emulsion is approx. 0.5 µm.

3. The lormetazepam-containing therapeutic composition, in particular a sedative according to claim 1 or 2 **characterised in that** the pH of the solution is adjusted to approx. 7.0 to approx. 8.0.

4. A process for the production of a lormetazepam-containing therapeutic composition, in particular a sedative according to one of claims 1 to 3, **characterised in that** an emulsion of soybean oil, glycerol and egg yolk phospholipids is formed with a quantity of water sufficient for injection purposes in such a way that an average droplet size of the emulsified phase of 0.5 µm is obtained,
that the pH of the emulsion is adjusted to 7.0 to 8.0 by means of a sodium hydroxide solution,
that, in a separate batch, lormetazepam is suspended as micronised powder in ethanol or another suitable alcohol,
that the lormetazepam-ethanol suspensions is mixed with the oil-in-water emulsion and that water is added in order to obtain an injectable solution.

5. The process according to claim 4 **characterised in that** the oil-in-water emulsion is produced in a high pressure process vessel under a nitrogen atmosphere and at temperatures of approx. 20 °C to approx. 30 °C.

6. The process according to claim 4 or 5 **characterised in that** the oil-in-water emulsion is filtered after the last mixing step in order to separate off particles more than 1,0 µm in size.

## Revendications

1. Composition thérapeutique contenant du lormétazépam, en particulier sédatif, pour administration intraveineuse, **caractérisée en ce que** le lormétazépam est introduit dans une émulsion huile dans eau, l'émulsion huile dans eau étant constituée d'un mélange d'huile de soja, de glycérol et de phospholipides de jaune d'oeuf avec une quantité suffisante d'eau pour injection.

2. Composition thérapeutique contenant du lormétazépam, en particulier sédatif, selon la revendication 1, **caractérisée en ce que** la taille moyenne des gouttes de l'émulsion est d'environ 0,5 µm.

3. Composition thérapeutique contenant du lormétazépam selon la revendication 1 ou la revendication 2, **caractérisée en ce que** le pH de la solution est établi entre environ 7,0 et environ 8,0.

4. Méthode de fabrication d'une composition thérapeutique contenant du lormétazépam, en particulier sédatif, selon l'une des revendications 1 à 3, **caractérisée en ce qu'**une émulsion d'huile de soja, de glycérol et de phospholipide de jaune d'oeuf est formée avec une quantité suffisante d'eau pour injection, de manière à obtenir une taille moyenne des gouttes de la phase émulsifiée de 0,5 µm, **en ce que** le pH de l'émulsion est établi entre 7,0 et 8,0 avec une solution d'hydroxyde de sodium, **en ce que** dans une formulation distincte, le lormétazépam sous forme de poudre micronisée est en mis en suspension dans de l'éthanol ou un autre alcool adéquat, **en ce que** la suspension lormétazépam - éthanol est mélangée avec l'émulsion huile dans eau et **en ce que** de l'eau est ajoutée afin d'obtenir une solution injectable.

5. Méthode selon la revendication 4 **caractérisée en ce que** l'émulsion huile dans eau est fabriquée dans un récipient sous haute pression sous atmosphère d'azote et à des températures d'environ 20 °C à environ 30 °C.

6. Méthode selon la revendication 4 ou la revendication 5 **caractérisée en ce que** l'émulsion huile dans eau de la dernière étape de mélange est filtrée afin de séparer les particules de plus de 1,0 µm.
